Europäisches Patentamt

European Patent Office

Office européen des brevets

(11) Veröffentlichungsnummer: **0 318 825**

**A2**

(12) **EUROPÄISCHE PATENTANMELDUNG**

(21) Anmeldenummer: **88119546.5**

(22) Anmeldetag: **24.11.88**

(51) Int. Cl.⁴: **C07C 99/00 , C07C 101/62**

(30) Priorität: **03.12.87 CH 4720/87**

(43) Veröffentlichungstag der Anmeldung:
**07.06.89 Patentblatt 89/23**

(84) Benannte Vertragsstaaten:
**AT BE CH DE FR GB IT LI NL**

(71) Anmelder: **F. HOFFMANN-LA ROCHE & CO.
Aktiengesellschaft**

**CH-4002 Basel(CH)**

(72) Erfinder: **Guerrato, Alfredo
Via Paninsacco 4
I-36070 Trissino (VI)(IT)**
Erfinder: **Karpf, Martin
In den Haselmatten 4
CH-4153 Reinach(CH)**
Erfinder: **Kjellsaa-Berger, Hanny
Eggfluhweg 1
CH-4147 Aesch(CH)**
Erfinder: **Kompis, Ivan
Goldentalweg 16
CH-4104 Oberwil(CH)**
Erfinder: **Schlageter, Markus
Nussbaumweg 24
CH-4103 Bottmingen(CH)**

(74) Vertreter: **Grossner, Lutz, Dr. et al
Grenzacher Strasse 124 Postfach 3255
CH-4002 Basel(CH)**

㉒ Verfahren zur Herstellung eines Benzoesäureesters.

㉗ 4-Dimethylamino-3,5-dimethoxybenzoesäuremethylester, ein Zwischenprodukt bei der Herstellung des antibakteriell wirksamen Aditoprims, erhält man aus 4-Amino-3,5-dibrombenzoesäure durch Austausch der Bromatome gegen Methoxygruppen und nachfolgende Methylierung.

## Verfahren zur Herstellung eines Benzoesäureesters

Die Erfindung betrifft ein neues Verfahren zur Herstellung des 4-(Dimethylamino)-3,5-dimethoxy-benzoesäuremethylesters, der als Zwischenprodukt zur Herstellung des 2,4-Diamino-5-(3,5-dimethoxy-4-dimethylamino)benzylpyrimidins (Aditoprim), einer antibakteriell wirksamen Verbindung, von Interesse ist.

Der wirtschaftlichen Verwertung von Aditoprim, insbesondere im veterinärmedizinischen Bereich standen bisher hohe Herstellungskosten für diese Verbindung entgegen, wenn bekannte Herstellungsverfahren wie z.B. die in der DE-Patentschrift Nr. 2 443 682 beschriebenen Verfahren angewandt werden sollten.

Das vorliegende Verfahren ermöglicht es, Aditoprim wesentlich kostenökonomischer herzustellen, als dies mit den bekannten Herstellungsverfahren möglich war. Dieses Resultat wird dadurch erreicht, dass ein Verfahren entwickelt wurde, bei dem p-Aminobenzoesäure zu 4-Amino-3,5-dibrombenzoesäure bromiert wird, diese in 4-Amino-3,5-dimethoxybenzoesäure überführt und danach zu 4-(Dimethyl amino)3,5-dimethoxybenzoesäuremethylester methyliert wird. Die letztere Verbindung kann zu 4-(Dimethylamino)-3,5-dimethoxybenzaldehyd reduziert werden, der in an sich bekannter Weise in Aditoprim übergeführt werden kann. Bei dieser Reaktionssequenz stösst die Umwandlung der 4-Amino-3,5-dibrombenzoesäure und die Methylierung der 4-Aminogruppe insofern auf Schwierigkeiten, als bei Anwendung herkömmlicher Arbeitsmethoden unbefriedigende Ausbeuten bzw. grössere Mengen unerwünschter Nebenprodukte erhalten werden.

Es wurde nun gefunden, das der Austausch der Bromatome gegen Methoxygruppen un überraschend guter Ausbeute verläuft, wenn 4-Amino-3,5-dibrombenzoesäure mit einem Alkalimethylat, wie Natriummethylat in Gegenwart von $Cu_2O$ und Dimethylformamid oder dimethylacetamid umgesetzt wird. Weiterhin wurd gefunden, dass die Methylierung der so erhaltenen 4-Amino-3,5-dimethoxybenzoesäure mittels Dimethylsulfat in Gegenwart einer Base, wie einem Alkalicarbonat oder -hydrogencarbonat, z.B. Kaliumcarbonat oder Natriumhydrogencarbonat in überraschend hoher Ausbeute verläuft, wenn als Lösungsmittel ein niederes aliphatisches oder cycloaliphatisches Keton verwendet wird.

Das erfindungsgemässe Verfahren zur Herstellung des 4-(Dimethylamino)-3,5-dimethoxybenzoesäuremethylesters ist somit dadurch gekennzeichnet, dass man 4-Amino-3,5-dibrombenzoesäure mit einem Alkalimethylat in Gegenwart von $Cu_2O$ und Dimethylformamid oder Dimethylacetamid zu 4-Amino-3,5-dimethoxybenzoesäure umsetzt und diese mit Dimethylsulfat in Gegenwart einer Base in einem niederen aliphatischen oder cycloaliphatischen Keton unter Erwärmen methyliert.

In der ersten Verfahrensstufe wird eine etwa molare Menge $Cu_2O$, bezogen auf das Ausgangsmaterial, 4-Amino-3,5-dibrombenzoesäure, eingesetzt. Als Alkalimethylat wird vorzugsweise Natriummethylat in Methanol, zweckmässig in grösserem Ueberschuss z.B. in bis zu 10-facher molarer Menge, bezogen auf das Ausgangsmaterial, eingesetzt. Weiterhin wird das Dimethylformamid oder das Dimethylacetamid in grösserem Ueberschuss, z.B. in etwa 10- bis 20-facher molarer Menge, bezogen auf das Ausgangsmaterial, zugesetzt. Dimethylformamid bzw. Dimethylacetamid wirken als N-Acylierungsmittel, wobei die intermediär gebildete N-Formyl- bzw. N-Acetylgruppe im basischen Milieu im Zuge der Aufarbeitung des Reaktionsgemischs wieder abgespalten wird. Die Reaktion wird unter Erwärmen durchgeführt. Zweckmässig erwärmt man bis zum Siedepunkt des Reaktionsgemisches und destilliert den grösseren Teil der Lösungsmittel bei Atmosphärendruck ab. Der Rückstand wird zur Aufarbeitung mit Alkali, z.B. Natronlauge, erhitzt, abfiltriert, das Filtrat angesäuert (pH 5-6) und das Produkt durch Filtration oder Extraktion mit einem geeigneten Lösungsmittel isoliert, gewaschen und getrocknet.

In der zweiten Verfahrensstufe wird die erhaltene 4-Amino 3,5-dimethoxybenzoesäure mit Dimethylsulfat in Gegenwart einer Base, vorzugsweise Kaliumcarbonat, methyliert. Das Methylierungsmittel wird zweckmässig in etwa stöchiometrischer Menge eingesetzt. Beispiele von niederen aliphatischen und cycloaliphatischen Ketonen, die in dieser Stufe als Lösungsmittel verwendet werden, sind Aceton, Aethylmethylketon, Isopropylmethylketon, Isobutylmethylketon und Cyclohexanon. Bevorzugt sind Aceton und Aethylmethylketon. Die Reaktionstemperatur beträgt zweckmässig etwa 50-70° C. Das Reaktionsgemisch kann in üblicher Weise, z.B. durch Extraktion, aufgearbeitet werden.

Die nachstehenden Beispiele erläutern das Verfahren weiter.

### Beispiel 1

258 g Kupfer(I)oxid und 1670 ml Natriummethylatlösung (30% in Methanol) wurden unter Rühren und Argonbegasung 15 Minuten am Rückfluss gekocht (88-93° C). Bei dieser Temperatur wurde eine Lösung bestehend aus 531 g 3,5-Dibrom-4-aminobenzoesäure, 1670 ml Natriummethylatlösung (30% in Methanol) und 2700 ml Dimethylformamid (über Molekularsieb getrocknet) unter gleichzeitigem Abdestillieren zugetropft. Die Lösungsmittel wurden anschliessend weiter abdestilliert. Insgesamt wurden 2200 ml abdestilliert. Die entstandene rote Suspension wurde noch 45 Minuten bei 113-114°C weitergerührt, danach wurden im Wasserstrahlvakuum 1300 ml Dimethylformamid abdestilliert. Zu dem entstandenen roten Rückstand wurden 7500 ml 4N Natronlauge zugegeben, und die Suspension 30 Minuten bei ca. 100°C gerührt. Dabei wurden nochmals 800 ml abdestilliert. Die heisse Suspension wurde über eine Glassinternutsche abgenutscht, der Rückstand dreimal mit je 1800 ml Wasser heiss aufgeschlämmt und abfiltriert. Die auf 5°C abgekühlte Lösung wurde unter Rühren mit 2000 ml Essigsäure auf PH 5 gestellt. Die entstandene braune Suspension wurde genutscht und der Rückstand mit 1000 ml Eiswasser gewaschen. Der Rückstand wurde bei 60°C im Wasserstrahlvakuum getrocknet. Das so erhaltene Rohprodukt wurde mit 3200 ml Methanol aufgekocht, die braune Suspension genutscht und das Filtrat zur Trockene eingedampft. Man erhielt 313 g (88%) 4-Amino-3,5-dimethoxybenzoesäure.

## Beispiel 2

100 g 4-Amino-3,5-dimethoxybenzoesäure und 223 g Kaliumcarbonat wurden in 201 g Aethylmethylketon unter Rühren auf 70°C erwärmt und mit 15,2 ml Dimethylsulfat versetzt. Das Reaktionsgemisch wurde auf Rückflusstemperatur erhitzt (~80°C) und 1 Stunde bei dieser Temperatur gerührt. Danach wurden 10 ml Essigsäure zugegeben, das Reaktionsgemisch 30 Minuten bei ~80°C weitergerührt, auf 50°C abgekühlt, filtriert, der Rückstand mit 200 ml Aethylmethylketon (50°C) gewaschen und die vereinigten Filtrate eingedampft. Man erhielt 117 g Rohprodukt, das aus der 6-7-fachen Menge Methanol/Wasser (1:1) unter Zusatz von 10 g Kohle kristalli siert, 95-100 g (79-84%) 4-Dimethylamino-3,5-dimethoxybenzoesäuremethylester, Schmelzpunkt 65-69°C, lieferte.

## Beispiel 3

10 g 4-Amino-3,5-dimethoxybenzoesäure und 24,5 g Kaliumcarbonat werden in 65 ml Aceton unter Rühren auf 50°C erwärmt und mit 17 ml Dimethylsulfat versetzt. Das Reaktionsgemisch wird 5 Stunden bei dieser Temperatur gerührt. Nach Abkühlen des Reaktionsgemisches auf 30°C werden 50 ml Wasser, gefolgt von 80 ml 10-Proz. Salzsäure dazugefügt. Das Reaktionsgemisch wird dreimal mit je 100 ml Isopropylacetat extrahiert. Die organische Phase wird verworfen, die wässrige Phase wird mit 4 ml 28-proz. Natronlauge auf pH 9 gestellt und dreimal mit je 100 ml Isopropylacetat extrahiert. Die organische Phase wird zweimal mit je 100 ml Wasser gewaschen, mit Magnesiumsulfat getrocknet und am Rotationsverdampfer eingeengt. Das Rohprodukt (14 g) wird aus 150 ml Hexan umkristallisiert und ergibt 8,8 g (73%) 4-Dimethylamino-3,5-dimethoxybenzoesäuremethylester. Schmelzpunkt 72,5-74°C.

## Ansprüche

1. Verfahren zur Herstellung des 4-(Dimethylamino)-3,5-dimethoxybenzoesäuremethylesters, dadurch gekennzeichnet, dass man 4-Amino-3,5-dibrombenzoesäure mit einem Alkalimethylat in Gegenwart von $Cu_2O$ und Dimethylformamid oder Dimethylacetamid zu 4-Amino 3,5-dimethoxybenzoesäure umsetzt und diese mit Dimethylsulfat in Gegenwart einer Base in einem niederen aliphatischen oder cycloaliphatischen Keton unter Erwärmen methyliert.

2. Verfahren nach Anspruch 1, dadurch gekennzeichnet, dass man die Methylierung in Aceton oder Aethylmethylketon durchführt.